(19) **Europäisches Patentamt**

**European Patent Office**

**Office européen des brevets**

(11) Veröffentlichungsnummer: **0 138 089**
**B1**

(12) **EUROPÄISCHE PATENTSCHRIFT**

(45) Veröffentlichungstag der Patentschrift:
11.05.88

(21) Anmeldenummer: **84111158.6**

(22) Anmeldetag: **19.09.84**

(51) Int. Cl.⁴: **A 61 M 29/00, A 61 M 29/02**

㉞ Bougie.

(30) Priorität: 29.09.83 DE 8328036 U

(43) Veröffentlichungstag der Anmeldung:
24.04.85 Patentblatt 85/17

(45) Bekanntmachung des Hinweises auf die Patenterteilung:
11.05.88 Patentblatt 88/19

(84) Benannte Vertragsstaaten:
DE FR GB SE

(56) Entgegenhaltungen:
DE - A - 2 851 547
US - A - 2 541 691
US - A - 3 918 456
US - A - 4 122 591
US - A - 4 195 624
US - E - 30 365

(73) Patentinhaber: **Koss, Walter, Industriestrasse,
D-6222 Geisenheim/Rhein (DE)**

(72) Erfinder: **Buess, Gerhard, Dr., Hubertushof,
D-5024 Pulheim (DE)**
Erfinder: **Koss, Walter, Industriestrasse,
D-6222 Geisenheim/Rhein (DE)**

(74) Vertreter: **Blumbach Weser Bergen Kramer Zwirner
Hoffmann Patentanwälte, Sonnenbergerstrasse 43,
D-6200 Wiesbaden 1 (DE)**

## Beschreibung

Die Erfindung betrifft ein Bougie zur Dehnung der Speiseröhre, der Luftwege und anderer Hohlorgane oder Körperöffnungen mit einem Querschnittsänderungen aufweisenden Schlauch zur Aufnahme eines Endoskops.

Zum Bougieren der Speiseröhre, beispielsweise bei Stenosen, die durch ein Carzinom hervorgerufen werden, oder auch bei Stenosen anderer Ursache werden in bekannter Weise harte Drähte mit aufgeschraubten, olivenförmigen Verdickungen verwendet, von denen gegebenenfalls auch mehrere angebracht sind. Mit den Verdickungen erweitert man beim Durchstossen die Speiseröhre im Bereich der Stenose, bis mit der jeweils grössten Olive die erwünschte Dehnung und Erweiterung erreicht ist. Der Nachteil dieses Verfahrens besteht darin, dass zur genauen Verfolgung und Kontrolle nur eine röntgenologische Sicht mit entsprechender Strahlenbelastung des Patienten möglich ist. Man hat in der Praxis auch bereits versucht, ein Endoskop zum Bougieren einzusetzen, indem ein starrer, mit stufenförmigen Verdickungen versehener Schlauch über das Endoskop geschoben wird. Mit der steuerbaren Spitze des Endoskops, die über Drähte in beliebige Richtungen und auch nach rückwärts gebogen werden kann, lässt sich dann das Bougieren verfolgen. Nachteilig ist hierbei jedoch, dass das sehr teure Endoskop insbesondere durch die Vorderkante des Bougierrohres beschädigt werden kann und Längsverschiebungen des Endoskops im Rohr nicht sicher vermieden sind. Darüber hinaus lässt die Abdichtung zwischen dem Rohr und dem Endoskop zu wünschen übrig.

Bekannt ist auch schon ein Gallengang-Katheter (US-A 3 918 456) mit einem glatten Schlauch, dessen vorderes Ende steifer ausgebildet ist und eine perlenförmige Verdickung mit dem Durchmesser eines Gallengangs besitzt. Am hinteren Ende des Katheters ist ein becherförmiges Endstück zum Einführen einer Injektionsspritze angeordnet.

Der Erfindung liegt demgemäss die Aufgabe zugrunde, ein Bougie zur Verwendung in Verbindung mit Endoskopen zu schaffen, das nach dem Einschieben des Endoskops sicher festgelegt ist, Beschädigungen des empfindlichen und teuren Endoskops vermeidet und für eine gute Abdichtung insbesondere am vorderen Ende des Schlauchs sorgt. Die Lösung der Aufgabe ist im Patentanspruch 1 angegeben.

Das weiche und schmiegsame Ende des Schlauches ermöglicht ein müheloses Durchschieben des im allgemeinen etwas dickeren Biegeabschnittes des Endoskops, der die Steuerbewegungen ermöglicht, wobei nach dem Durchtreten dieses dickeren Abschnittes trotzdem ein gutes und dichtendes Anliegen zwischen dem Schlauchende und dem Endoskop erzielt wird. Das gilt auch für Endoskope mit abweichendem Durchmesser, so dass nicht für jedes Endoskop ein besonderes Bougie erforderlich ist. Der Kopf des Endoskops, der die verschiedenen Anschlüsse, die Beleuchtungseinrichtungen und den Einblick enthält, kann so in das becherförmige Ende eingedrückt werden, dass eine sichere Verklemmung erreicht wird und keine Axialbewegungen zwischen dem Schlauch und dem Endoskop auftreten können. Der glatte Schlauch kann ohne Schwierigkeiten in seiner Länge so angepasst sein, dass nach dem Eindrücken des Endoskopkopfes in das becherförmige Endstück die Spitze des Endoskops mit der gewünschten Länge aus dem Schlauch austritt.

Weiterbildungen der Erfindung sind Gegenstand der Unteransprüche. So können die Querschnittsänderungen in an sich bekannter Weise (GB-A 2 023 009) aus abwechselnd kegelförmig dicker werdenden und gleichbleibenden Abschnitten bestehen. Zusätzlich zu diesen Verdickungen oder anstelle der Verdickungen kann auch in an sich bekannter Weise (US-E 30 365) vorgesehen sein, dass der steifere Abschnitt einen Balg aufweist, der mittels eines parallel zum Bougie geführten Schlauches aufblähbar ist. Der glatte Schlauch kann zur Befestigung an dem steiferen Abschnitt und/oder den Schlauch und/oder einen rohrförmigen Ansatz des Endstücks angebracht oder in diese Teile eingearbeitet sein. Eine homogene Verbindung lässt sich an den Einführungsstellen beispielsweise durch Kleben oder Ultraschallschweissen erzielen. Es kann auch zweckmässig sein, die Verbindung zwischen dem Endstück und dem glatten Schlauch leicht lösbar zu gestalten, um beispielsweise eine Längenanpassung des Bougie an das Endoskop durch Absetzen des Schlauches zu ermöglichen. Die lösbare Verbindung kann eine Steckverbindung oder auch eine Schraubverbindung sein.

Der rohrförmige Ansatz des Endstücks kann in weiterer Ausbildung der Erfindung eine dem Schlauchaussendurchmesser angepasste Einstecköffnung aufweisen, an die sich eine dem Schlauchinnendurchmesser angepasste Bohrung anschliesst, derart, dass das Innere der Bohrung glatt in das Schlauchinnere übergeht. Das Endoskop lässt sich dann leicht einschieben, weil keine hindernde Aufsetzkante am Schlauchende auftritt. Zur Erleichterung der Handhabung kann das becherförmige Endstück mit einem Abschlussflansch versehen sein. Zur besseren Anpassung an unterschiedliche Endoskopköpfe kann in das Endstück ein Ring eingepresst sein. Gegebenenfalls lassen sich auch mehrere Ringe verwenden. Die Ringe können kreisförmig, oval oder auch anders geformt sein. Ihre Öffnungen können in Anpassung an den Endoskopkopf ebenfalls unterschiedliche Formen annehmen.

Für sogenannte Null-Stenosen, bei denen praktisch kein Durchgang mehr vorhanden ist, empfiehlt eine Weiterbildung der Erfindung eine auf das vordere Ende des Endoskops aufschiebbare Kappe mit dünn auslaufendem Ende. Das dünne oder spitze Ende der Kappe ermöglicht dann das Einschieben in die Stenose und das anschliessende Aufweiten. Nach Durchtritt durch die Stenose soll zweckmässig die Kappe entfernt werden, um die Beweglichkeit des Endoskops und die Sicht nicht zu behindern. Dazu kann zweckmässig die Kappe entweder aus verdaubarem Material bestehen, das sich im Magen- oder Darmsaft bzw. einer anderen Körperflüssigkeit auflöst, oder die Kappe kann aus einem elastomeren Kunststoff mit einer für die medizinische Verwendung geeigneten Qualität bestehen und mittels der im Endoskop vorhandenen Instrumente abstossbar sein. Im übrigen wird die Kappe zweckmässig aus möglichst durchsichtigem Material hergestellt, um wenigstens eine gewisse Sichtkontrolle vor dem Auflösen bzw. Abstossen der Kappe zu haben.

Das Bougie nach der vorliegenden Erfindung lässt sich in Weiterbildung auch dazu verwenden, einen Endotubus unter Sichtkontrolle zu setzen. Dazu wird empfohlen, dass der steifere Abschnitt ein Endotubus ist, der am rückwärtigen Ende einen Aufnahmebecher für den Schlauch und am vorderen Ende einen das Zurückziehen erschwerenden sich nach rückwärts erweiternden Kragen an sich bekannter Art besitzt. Der Aufnahmebecher sorgt dafür, dass der Endotubus vom Endoskop geführt wird, dabei aber der Schlauch Druckkräfte aufnimmt, das Endoskop also entlastet ist. Zwischen dem Schlauchende und dem Becher kann eine leicht lösbare Verbindung vorgesehen sein, beispielsweise in Form eines Bajonettverschlusses, der durch Verdrehen gelöst wird, oder in Form eines Einschnappverschlusses.

Nachfolgend wird die Erfindung anhand der Zeichnung beschrieben. Es zeigen:

Fig. 1 eine schematische Ansicht eines Ausführungsbeispiels;

Fig. 2 und Fig. 2a die schematische Ansicht eines weiteren Ausführungsbeispiels;

Fig. 3 eine vergrösserte Schnittdarstellung des becherförmigen Endstücks gemäss Fig. 1 oder 2;

Fig. 4 ein weiteres Ausführungsbeispiel der Erfindung;

Fig. 5 eine vergrösserte, teilweise geschnittene Ansicht des Bougieendes mit einer aufgeschobenen Kappe.

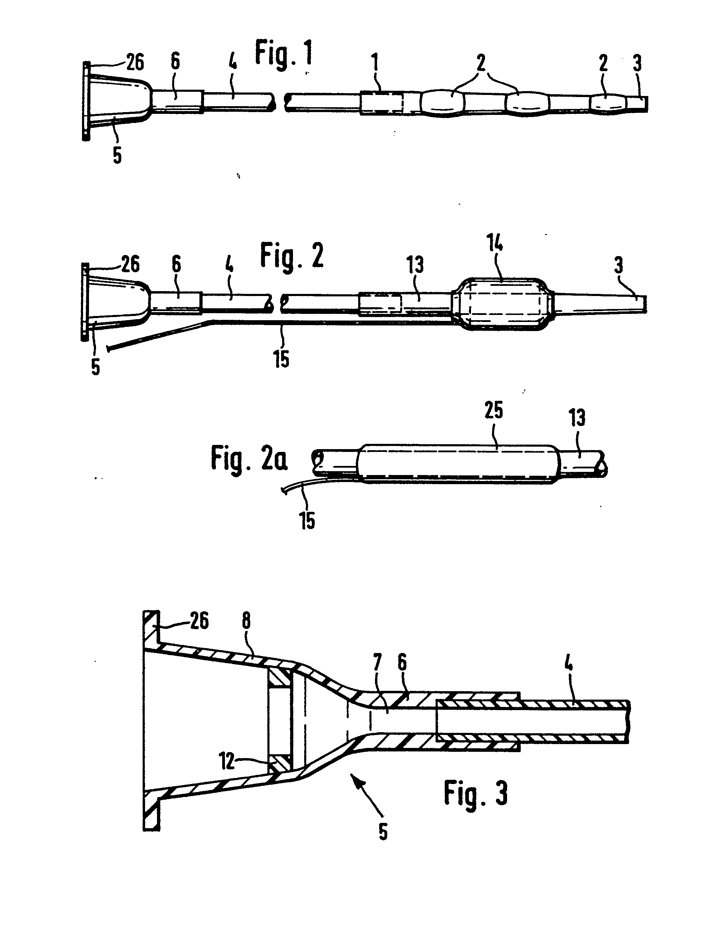

Das in Fig. 1 dargestellte Ausführungsbeispiel weist am vorderen, dem Körper des Patienten zugewandten Ende den eigentlichen Bougierabschnitt 1 mit stufenförmig grösser werdenden Verdickungen 2 auf. Das vordere Ende 3 des Bougierabschnittes 1 ist weich, dehnbar und anschmiegsam ausgebildet, so dass ein eingeschobenes Endoskop (nicht dargestellt) mit seinem etwas dickeren, vorderen Biegerabschnitt leicht durchgeschoben werden kann und eine gute Anschmiegung und Abdichtung zwischen dem Ende 3 und dem Endoskopschaft erzielbar ist. An den Bougierabschnitt 1 schliesst sich ein glatter Schlauch 4 an, der homogen mit dem Abschnitt 1 verbunden ist. Hierzu wird der Schlauch 4 entsprechend der gestrichelten Darstellung in den Abschnitt 1 eingeschoben und verklebt oder verschweisst. Am anderen Ende ist der Schlauch 4 entsprechend der genaueren Schnittdarstellung in Fig. 3 in ein becherförmiges Endstück 5 mit einem Ansatz 6 eingesteckt. Die Einstecköffnung nimmt dabei den Schlauch 4 so auf, dass sich das Schlauchinnere ohne Absatz in eine Bohrung 7 im Ansatz 6 fortsetzt. Ein konisch zulaufender Becher 8 des Endstücks 5 nimmt den jeweiligen Kopf oder Halter des Endoskops (nicht dargestellt) und/oder auch das üblicherweise durch eine konisch zulaufende Verstärkung geschützte Ende des Endoskopstabes klemmend auf, wobei durch die konische Form und Flexibilität eine Anpassung an unterschiedliche Ausführungsformen erreicht wird. Ein Flansch 26 dient als Griffrand und erleichtert die Handhabung. Ein Ring 12 ist in das Endstück 5 eingepresst, wodurch eine Anpassung an abweichende Kopfformen von Endoskopen möglich ist. Gegebenenfalls können auch mehrere oder unterschiedliche Ringe dieser Art eingesetzt sein.

Das Ausführungsbeispiel gemäss Fig. 2 entspricht dem Ausführungsbeispiel gemäss Fig. 1 mit der Ausnahme, dass anstelle des steiferen Abschnittes 1 ein Rohr 13 aufgesetzt ist, das einen aufblähbaren Balg 14 enthält, der mit dem Rohr 13 homogen durch Schweissen oder Kleben verbunden ist. Ein nur schematisch dargestellter Schlauch 15 führt aus dem Balg 14 am Bougie entlang nach aussen und ermöglicht so die Erweiterung von Stenosen durch Aufblähen des Balges mittels Luft oder einer Flüssigkeit. Der Schlauch 15 kann auch integraler Teil des Rohres 13 und/oder des Schlauches 4 sowie des Ansatzes 6 sein und gegebenenfalls auch von innen in den Balg 14 führen. Fig. 2a zeigt eine Abwandlung des Ausführungsbeispiels nach Fig. 2. Um das Rohr 13 ist ein dünnwandiges Blährohr 25 aus elastomerem Kunststoffmaterial eng anliegend angebracht und an seinen Enden homogen mit dem Rohr 13 verbunden. Durch das enge Anliegen im Ruhezustand lässt sich das Bougie leicht einführen. Das Aufblähen kann beispielsweise bis zu einem Durchmesser von etwa 40 mm erfolgen.

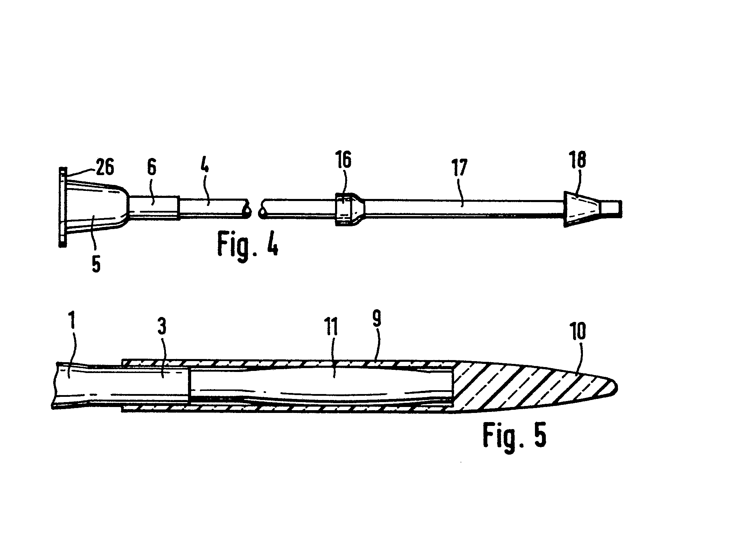

Das Ausführungsbeispiel gemäss Fig. 4 weicht von den Ausführungsbeispielen nach Fig. 1 und 2 dadurch ab, dass an den Schlauch 4 mittels eines Aufnahmebechers 16 ein rohrförmiger Endotubus 17 angesetzt ist, der am vorderen Ende in an sich bekannter Weise einen sich nach rückwärts erweiternden Kragen 18 besitzt, der nach dem Durchschieben durch eine Verengung ein Zurückgleiten verhindert. Mit dem Bougie gemäss Fig. 4 kann daher unter Sichtkontrolle mittels des durchgeführten Endoskops der Endotubus gesetzt werden, wobei zusätzlich das Endoskop (nicht gezeigt) eine Führung und Versteifung bewirkt. Zwischen dem Schlauch 4 und dem Becher 16 kann statt der dargestellten Steckverbindung beispielsweise eine Bajonettverbindung (nicht dargestellt) vorgesehen sein, die durch Verdrehen nach dem Einsetzen des Endotubus 17 lösbar ist.

Alle Teile des Bougie bestehen aus einem elastomeren Kunststoff. Das Material der Spitze 3 des Bougierabschnittes 1 ist dabei, wie bereits erwähnt, aus weich eingestelltem Material hergestellt. Der übrige Teil des Bougierabschnittes 1 ist ebenso wie der Schlauch 4 härter eingestellt, derart, dass das Endoskop auch bei Ausübung von Druckkräften zur Überwindung einer Stenose nicht gestaucht wird.

Gemäss Fig. 5 ist auf das weiche Ende 3 des Bougierabschnittes 1 und den dort austretenden, verdickten Biegeabschnitt 11 des Endoskops eine geschnitten dargestellte Kappe 9 mit einer abgerundeten Spitze 10 aufgeschoben. Die Kappe 9, die aus durchsichtigem Kunststoff mit einer für medizinische Zwecke geeigneten Qualität besteht, stützt sich an der Frontfläche des Endoskops ab, so dass die Kappe unter Druckausübung ihrer Spitze 10 auch noch durch sehr enge oder Null-Stenosen geschoben werden kann. Nach Durchtritt durch die Stenose lässt sich die Kappe 9 mittels der üblicherweise im Endoskop untergebrachten Instrumente (nicht dargestellt) abstossen und verlässt dann den Körper durch den Darm. Gegebenenfalls kann die Kappe 9 auch nur auf das Endoskop 11 aufgesetzt sein.

**Patentansprüche**

1. Bougie zur Dehnung der Speiseröhre, der Luftwege und anderer Hohlorgane oder Körperöffnungen mit einem Querschnittsänderungen aufweisenden Schlauch zur Aufnahme eines Endoskops, dadurch gekennzeichnet, dass das vordere, zum Körper gerichtete Ende (3) des Schlauchs (1, 4) weich, dehnbar und schmiegsam ausgebildet ist, derart, dass ein Endoskop mit seinem vorderen Biegeabschnitt leicht durchschiebbar ist und eine gute Anschmiegung und Abdichtung zwischen dem vorderen Ende (3) und dem Endoskopschaft erzielbar ist, dass sich daran ein steiferer Abschnitt (1, 13, 17) mit den Querschnittsveränderungen (2, 14, 18) und dann ein glatter Schlauch (4) anschliessen, an dessen freiem Ende ein becherförmiges, innen in Richtung zum Schlauch (4) konisch sich verjüngendes Endstück (5) zur Aufnahme eines Endoskopkopfes angeordnet ist, und dass der steifere Abschnitt (1, 13, 17) und der glatte Schlauch (4) härter als das zum Körper gerichtete Ende (3) sind, derart, dass ein eingeschobenes Endoskop auch bei Ausübung von Druckkräften nicht gestaucht wird.

2. Bougie nach Anspruch 1, dadurch gekennzeichnet, dass die Querschnittsänderungen (2) aus abwechselnd kegelförmig dicker werdenden und gleichbleibenden Abschnitten bestehen.

3. Bougie nach Anspruch 1, dadurch gekennzeichnet, dass der steifere Abschnitt (13) einen Balg (14) aufweist, der mittels eines parallel zum Bougie geführten Schlauches (15) aufblähbar ist.

4. Bougie nach Anspruch 1, 2 oder 3, dadurch gekennzeichnet, dass der glatte Schlauch (4) in den steiferen Abschnitt (1) einerseits und einen rohrförmigen Ansatz (6) des Endstückes (5) andererseits eingeführt ist.

5. Bougie nach Anspruch 4, dadurch gekennzeichnet, dass der glatte Schlauch (4) mit dem steiferen Abschnitt (1) homogen verbunden ist.

6. Bougie nach Anspruch 4 oder 5, dadurch gekennzeichnet, dass der glatte Schlauch (4) mit dem Endstück (5) homogen verbunden ist.

7. Bougie nach Anspruch 4 oder 5, dadurch gekennzeichnet, dass der glatte Schlauch (4) lösbar mit dem Endstück (5) verbunden ist.

8. Bougie nach einem der Ansprüche 4 bis 7, dadurch gekennzeichnet, dass der rohrförmige Ansatz (6) eine dem Schlauchaussendurchmesser angepasste Einsecköffnung aufweist, an die sich eine dem Schlauchinnendurchmesser angepasste Bohrung (7)) anschliesst, derart, dass das Innere der Bohrung (7) glatt in das Schlauchinnere übergeht.

9. Bougie nach einem der Ansprüche 1 bis 8, dadurch gekennzeichnet, dass das becherförmige Endstück (5) einen Abschlussflansch (26) aufweist.

10. Bougie nach einem der Ansprüche 1 bis 9, dadurch gekennzeichnet, dass in das becherförmige Endstück (5) ein Ring (12) eingepresst ist.

11. Bougie nach einem der Ansprüche 1 bis 10, gekennzeichnet durch seine Herstellung aus elastomerem Kunststoff.

12. Bougie nach einem der Ansprüche 1 bis 11, gekennzeichnet durch eine auf das vordere Ende (3) des Schlauches (4) aufschiebbare Kappe (9) mit dünn auslaufendem Ende (10).

13. Bougie nach Anspruch 12, dadurch gekennzeichnet, dass die Kappe (9) aus verdaubarem Material besteht.

14. Bougie nach Anspruch 12, dadurch gekennzeichnet, dass die Kappe (9) aus elastomerem Kunststoff besteht und abstossbar ausgebildet ist.

15. Bougie nach einem der Ansprüche 12 bis 14, dadurch gekennzeichnet, dass die Kappe (9) aus durchsichtigem Material besteht.

16. Bougie nach einem der Ansprüche 1 bis 11, dadurch gekennzeichnet, dass der steifere Abschnitt ein Endotubus (17) ist, der am rückwärtigen Ende einen Aufnahmebecher (16) für den Schlauch (4) und am vorderen Ende einen das Zurückziehen erschwerenden, sich nach rückwärts erweiternden Kragen (18) besitzt.

**Claims**

1. A bougie for dilating the esophagus, respiratory tracts and other hollow members or body orifices, the bougie comprising a variable-cross-section flexible tube adapted to receive an endoscope, characterised in that the tube front end (3) extending towards the body is soft, stretchable and pliant so that the front bendable part of an endoscope can readily be pushed through and an intimate engagement and satisfactory sealing tightness can be produced between the tube front end (3) and the endoscope stem, the tube front end (3) merges into a more rigid part (1, 13, 17) having the cross-sectional variations (2, 14, 18) and the latter part merges into a plain tube (4) having disposed at its free end a beaker-shaped end member (5) which narrows conically inwards towards the tube (4) and which is adapted to receive an endoscope head, and the more rigid part (1, 13, 17) and the plain tube (4) are harder than the tube end (3) near the body, so that an inserted endoscope is not upset even when subjected to pressures.

2. A bougie according to claim 1, characterised in that the cross-sectional variations (2) are in the form of alternately conically thickening parts and constant parts.

3. A bougie according to claim 1, characterised in that the more rigid part (13) has bellows (14) inflatable by means of a flexible tube (15) which extends parallel to the bougie.

4. A bougie according to claim 1 or 2 or 3, characterised in that the plain tube (4) is introduced at one end into the more rigid part (1) and at the other end into a tubular extension (6) of the end member (5).

5. A bougie according to claim 4, characterised in that the plain tube (4) is homogeneously connected to the more rigid part (1).

6. A bougie according to claim 4 or 5, characterised in that the plain tube (4) is homogeneously connected to the end member (5).

7. A bougie according to claim 4 or 5, characterised in that the plain tube (4) is releasably connected to the end member (5).

8. A bougie according to any of claims 4–7, characterised in that the tubular extension (6) is formed with an insertion orifice which is adapted to tube outer diameter and which is followed by a bore (7) adapted to tube internal diameter so that the interior of the bore (7) merges smoothly into the interior of the tube.

9. A bougie according to any of claims 1–8, characterised in that the beaker-shaped end member (5) has a closure flange (26).

10. A bougie according to any of claims 1–9, characterised in that a ring (12) is pressed into the beaker-shaped end member (5).

11. A bougie according to any of claims 1–10, characterised in that it is made of an elastomeric plastics.

12. A bougie according to any of claims 1–11, characterised by a cap (9) which can be pushed on to the tube front end (3) and which has an end (10) with a thin run-out.

13. A bougie according to claim 12, characterised in that the cap (9) is made of digestible material.

14. A bougie according to claim 12, characterised in that the cap (9) is made of an elastomeric plastics and is expellable.

15. A bougie according to any of claims 12–14, characterised in that the cap (9) is made of a transparent material.

16. A bougie according to any of claims 1–11, characterised in that the more rigid part is an endotube (17) having a receiving beaker (16) for the tube (4) at its rear end and, at its front end, a collar (18) which makes withdrawal difficult and which widens rearwardly.

## Revendications

1. Sonde pour l'élargissement du tube digestif, des voies respiratoires et autres organes creux ou orifices du corps, avec un tuyau souple présentant des variations de section transversale destiné à recevoir un endoscope, caractérisée en ce que l'extrémité (3) antérieure du tuyau souple (1, 4) dirigée vers le corps est molle, extensible et flexible de façon qu'un endoscope peut être facilement poussé à travers par son segment flexible avant et que l'extrémité avant (3) et la tige de l'endoscope peuvent s'appliquer l'une contre l'autre de façon souple et étanche, qu'à la suite de cette extrémité se trouvent successivement un segment plus rigide (1, 13, 17) avec les changements de section transversale (2, 14, 18) puis un tuyau souple (4) lisse à l'extrémité libre duquel est disposé un élément terminal (5) en forme de coupe, se rétrécissant en forme de cône en direction du tuyau souple (4) destiné à recevoir une tête d'endoscope, et que le segment plus rigide (1, 13, 17) et le tuyau souple (4) lisse sont plus durs que l'extrémité (3) dirigée vers le corps, de sorte que, même sous l'effet de forces de pression, l'endoscope introduit n'est pas écrasé.

2. Sonde selon la revendication 1, caractérisée en ce que les changements de section transversale (2) sont formés par une alternance de segments s'épaississant en forme de cône et de segments de diamètre constant.

3. Sonde selon la revendication 1, caractérisée en ce que le segment (13) présente un soufflet (14) qui peut être gonflé au moyen d'un tuyau souple (15) s'étendant parallèlement à la sonde.

4. Sonde selon une quelconque des revendications 1, 2 ou 3, caractérisée en ce que le tuyau souple (4) lisse est introduit d'un côté dans le segment (1) plus rigide et de l'autre côté dans un appendice (5) tubulaire de l'élément terminal (5).

5. Sonde selon la revendication 4, caractérisée en ce que le tuyau souple (4) lisse est relié de façon homogène au segment (1) plus rigide.

6. Sonde selon la revendication 4 ou 5, caractérisée en ce que le tuyau souple (4) lisse est relié de façon homogène à l'élément terminal (5).

7. Sonde selon la revendication 4 ou 5, caractérisée en ce que le tuyau souple (4) lisse est relié de façon détachable à l'élément terminal (5).

8. Sonde selon une quelconque des revendications 4 à 7, caractérisée en ce que l'appendice (6) tubulaire possède une ouverture d'introduction adaptée au diamètre extérieur du tuyau souple à laquelle se raccorde un canal (7) adapté au diamètre intérieur du tuyau souple, de sorte que la partie intérieure du canal (7) est prolongée de façon régulière par la partie intérieure du tuyau souple.

9. Sonde selon une quelconque des revendications 1 à 8, caractérisée en ce que l'élément terminal (5) en forme de coupe comporte à son extrémité une fausse-bride (26).

10. Sonde selon une quelconque des revendications 1 à 9, caractérisée en ce qu'une bague (12) est forcée dans l'élément terminal (5) en forme de coupe.

11. Sonde selon une quelconque des revendications 1 à 10, caractérisée en ce qu'elle est faite en matière plastique élastomère.

12. Sonde selon une quelconque des revendications 1 à 11, caractérisée par un capuchon (9) dont l'extrémité (10) est de forme effilée qui peut être engagé sur l'extrémité (3) avant du tuyau souple (4).

13. Sonde selon la revendication 12, caractérisée en ce que le capuchon (9) est fait en matériau pouvant être digéré.

14. Sonde selon la revendication 12, caractérisée en ce que le capuchon (9) est fait en matière plastique élastomère et peut être chassé.

15. Sonde selon une quelconque des revendications 12 à 14, caractérisée en ce que le capuchon (9) est fait en matériau transparent.

16. Sonde selon une quelconque des revendications 1 à 11, caractérisée en ce que le segment plus rigide est un endotube (17) qui possède à son extrémité postérieure une coupe de réception (16) pour le tuyau souple (4) et à son extrémité antérieure un collet (18) allant en s'élargissant vers l'arriere qui s'oppose au retrait.

Fig. 1

Fig. 2

Fig. 2a

Fig. 3

Fig. 4

Fig. 5